(19) Europäisches Patentamt
European Patent Office
Office européen des brevets

(11) Veröffentlichungsnummer: **0 237 480 B1**

(12) **EUROPÄISCHE PATENTSCHRIFT**

(45) Veröffentlichungstag der Patentschrift: **10.06.92**

(51) Int. Cl.5: **C07C 311/16**, C07C 309/29, C07C 309/86

(21) Anmeldenummer: **87810111.2**

(22) Anmeldetag: **27.02.87**

# (54) Verfahren zur Herstellung von 2-Alkoxybenzosulfonamiden.

(30) Priorität: **05.03.86 US 836288**

(43) Veröffentlichungstag der Anmeldung:
**16.09.87 Patentblatt 87/38**

(45) Bekanntmachung des Hinweises auf die Patenterteilung:
**10.06.92 Patentblatt 92/24**

(84) Benannte Vertragsstaaten:
**AT BE CH DE FR GB IT LI NL**

(56) Entgegenhaltungen:
**EP-A- 0 023 422**
**EP-A- 0 085 028**

(73) Patentinhaber: **CIBA-GEIGY AG**
**Klybeckstrasse 141**
**CH-4002 Basel(CH)**

(72) Erfinder: **Jaeggi, Franz-Josef**
**Oristalstrasse 42**
**CH-4410 Liestal(CH)**

EP 0 237 480 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues Verfahren zur Herstellung von 2-Alkoxybenzosulfonamiden.

Die nach dem neuen Verfahren herstellbaren 2-Alkoxybenzosulfonamide sind wertvolle Zwischenprodukte für die Synthese von herbiziden 2-Alkoxybenzosulfonyl-harnstoffen. Diese Klasse von hochwirksamen Herbiziden sind in der letzten Zeit in einer grossen Anzahl von Patentanmeldungen und Publikationen beschrieben worden. In den Europäischen Patentanmeldungen EP-A-23422, EP-A-44807 und EP-A-44808 sind die nach dem erfindungsgemässen Verfahren herstellbaren 2-Alkoxybenzosulfonamide, ihre Herstellung und ihre Verwendung im Syntheseverfahren für die herbiziden Endprodukte aus der Klasse der Sulfonylharnstoffe beschrieben.

Die bisher beschriebenen Herstellungsverfahren für Alkoxybenzosulfonamide sind für grosstechnische Anwendung weniger geeignet, weil entweder instabile Diazoniumsalze als Zwischenprodukte erzeugt werden und die Austauschreaktion vom Sandmeyer-Typus mit Cu(I)-Verbindungen nur einen unzureichenden Grad an Selektivität besitzt, oder weil Produktgemische entstehen, die aufwendige Trennungsmethoden erfordern.

Es besteht somit ein Bedürfnis nach einem kostengünstigen, im grosstechnischen Massstab durchführbaren Verfahren zur Herstellung von 2-Alkoxybenzosulfonamiden.

Ueberraschenderweise befriedigt das erfindungsgemässe neue Verfahren dieses Bedürfnis weitgehend.

Gemäss vorliegender Erfindung wird vorgeschlagen, die 2-Alkoxy-benzosulfonamide der Formel I

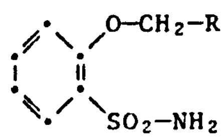

(I)

worin R für Chlormethyl, Methoxymethyl, $C_1$-$C_4$ Alkyl oder Trifluormethyl steht, in der Weise herzustellen, dass man ein 4-Alkoxy-chlorbenzol der Formel II

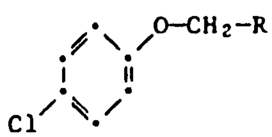

(II)

worin R die unter Formel I gegebene Bedeutung bat, mit Chlorsulfonsäure $ClSO_3H$ umsetzt, das entstandene Sulfonylchlorid der Formel III

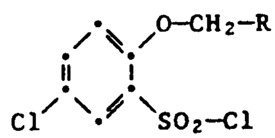

(III)

worin R die unter Formel I gegebene Bedeutung hat, mit Ammoniak in das Sulfonamid der Formel IV

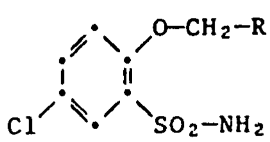

(IV)

worin R die unter Formel I gegebene Bedeutung hat, überführt und dieses in Gegenwart eines Edelmetallkatalysators mit Wasserstoff hydriert.

In der obigen Definition steht Alkyl für Methyl, Aethyl, n-Propyl, Isopropyl, n-Butyl, s-Butyl, Isobutyl oder tert.-Butyl.

Das Reaktionsprodukt der Formel I kann in an sich bekannter Weise direkt durch Reaktion mit einem

geeigneten Pyrimidinyl- oder Triazinylcarbamat oder einem entsprechenden Isocyanat zu einem Pflanzenwirkstoff aus der Klasse der Sulfonylharnstoffe umgesetzt werden. Als Alternative zu diesem Verfahren überführt man die 2-Alkoxybenzosulfonamide der Formel I zunächst im entsprechende Isocyanate oder Carbamate und setzt diese mit den geeigneten Pyrimidinyl- oder Triazinylaminen zu den pflanzenwirksamen Sulfonylharnstoffen um.

Die Ausgangsverbindungen der Formel II sind bekannt und können durch einfache Verätherungsreaktionen aus 4-Chlorphenol erzeugt werden.

Zur Durchführung des ersten Schrittes (II → III) des erfindungsgemässen Verfahrens verwendet man handelsübliche Chlorsulfonsäure. Zur Umsetzung werden pro Mol an Verbindung II mindestens 3 Mol Chlorsulfonsäure eingesetzt. Vorteilhaft ist die Verwendung eines grösseren Ueberschusses, beispielsweise von mindestens 5 Mol Chlorsulfonsäure pro Mol der Verbindung II. In einzelnen Ausführungsformen kann die Chlorsulfonsäure gleichzeitig als Reaktionspartner und als Lösungsmittel dienen. Im allgemeinen wird die Umsetzung (II → III) jedoch in einem inerten Lösungsmittel durchgeführt. Bewährte Lösungsmittel sind dafür Schwefelkohlenstoff, Toluol, Xylol, gesättigte Kohlenwasserstoffe wie Hexan, Dekan, Cyclohexan und chlorierte Kohlenwasserstoffe wie Methylenchlorid, Chloroform, Tetrachlorkohlenstoff, 1,2-Dichloräthan 1,2-Dichloräthylen, Tetrachloräthylen, Chlorbenzol oder Dichlorbenzol. Die bevorzugten Lösungsmittel sind Cyclohexan, n-Dekan, Methylenchlorid, 1,2-Dichloräthan und Chloroform. Die Reaktionstemperaturen liegen meistens zwischen -10° C und +100° C, vorzugsweise zwischen -10° C und +80° C.

In einer bevorzugten Ausführungsform der ersten Reaktionsstufe (II → III) stellt man die Verbindungen der Formel III her, indem man die entsprechende Verbindung der Formel II in einem inerten Lösungsmittel bei einer Temperatur zwischen -10° C und +100° C mit mindestens 3 Mol Chlorsulfonsäure pro Mol der Verbindung der Formel II umsetzt. Ganz besonders bevorzugt ist diejenige Ausführungsform, in welcher man die Reaktion (II → III) bei einer Temperatur zwischen -10° C und +80° C in Methylenchchlorid, 1,2-Dichloräthan oder Chloroform mit mindestens 5 Mol Chlorsulfonsäure pro Mol der Verbindung der Formel II durchführt.

Der erste Reaktionsschritt (II → III) kann auch unter Einsatz einer äquimolaren Menge an Chlorsulfonsäure durchgeführt werden, wenn man dabei einen zusätzlichen Reaktionsschritt in Kauf nimmt. Der wirtschaftliche und ökologische Vorteil besteht darin, dass man durch Verwendung einer äquimolaren Menge an Chlorsulfonsäure einen Ueberschuss an eingesetzter Chlorsulfonsäure weder aufarbeiten noch neutralisieren muss. Gemäss dieser Verfahrensvariante werden die Verbindungen der Formel II bei einer Temperatur zwischen 0° C und + 150° C, vorzugsweise zwischen +80° C und +60° C, mit einer äquimolaren Menge an Chlorsulfonsäure oder mit einem geringen Ueberschuss umgesetzt. Bei der Aufarbeitung erhält man in diesem Fall nicht direkt das Sulfonylchlorid der Formel III, sondern bei Neutralisation der Reaktionsmischung mit wässrigem Alkalihydroxid bei einer Temperatur zwischen 0° C oder +100° C, vorzugsweise zwischen 50° C oder 90° C, das entsprechende Sulfonsäure-alkalisalz der Formel V

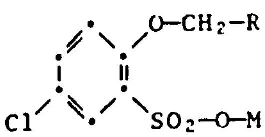

(V)

worin R die unter Formel I gegebene Bedeutung hat und M für ein Natrium oder Kaliumatom steht. Mit Vorteil wird diese Reaktion in einem inerten Lösungsmittel wie einem Alkan oder Chloralkan durchgeführt. In günstigen Fällen, z.B. wenn bei der gewählten Reaktionstemperatur alle Reaktionskomponenten flüssig sind, kann man auf ein Lösungsmittel verzichten und die Reaktion mit den reinen Reaktanden durchführen. Lösungsmittel der oben genannten Art sind Pentan, Hexan, Heptan, Oktan, Dekan, Dodecan, aber auch Cyclopentan oder Cyclohexan, sowie Methylenchlorid, Chloroform,, Tetrachlorkohlenstoff, Dichloräthan, Trichloräthan oder Tetrachloräthan. Bevorzugt sind Cyclohexan und n-Dekan.

Die Verbindung der Formel V wird mit üblichen Chlorierungsmitteln wie Phosphoroxychlorid, Thionylchlorid oder vorzugsweise Phosgen in das Sulfochlorid der Formel III umgewandelt. Vorteilhaft ist die Verwendung eines Katalysators wie Dimethylformamid oder Dimethylacetamid. Die Reaktionsbedingungen bei der Genese des Sulfochlorids aus der freien Säure, bzw. dem Alkalisalz (V → III) entsprechen den allgemein üblichen Bedingungen für diesen Reaktionstyp. Als wesentliches Merkmal seien Feuchtigkeitsausschluss sowie Reaktionsneutralität des Lösungsmittels genannt. Solche Bedingungen sind bei der direkten Sulfonylchloridherstellung (II → III) genannt. Bevorzugte Reaktionstemperaturen liegen zwischen 60° C und 120° C.

Die Ueberführung des Sulfonylchlorids der Formel III in das entsprechende Sulfonamid der Formel IV erfolgt unter den für diesen an sich bekannten Reaktionsschritt üblichen Bedingungen, beispielsweise durch Versetzen der Verbindung der Formel III mit einer wässrigen Ammoniak-Lösung unter Normaldruck und bei einer Temperatur zwischen 0°C und +100°C, vorzugsweise 0°C und +30°C; oder durch Behandlung einer Verbindung der Formel III in einem inerten Lösungsmittel, gegebenenfalls in Gegenwart eines säurebindenden Mittels, mit Ammoniak. Lösungsmittel und säurebindende Mittel sind beispielsweise: Carbonate wie Natrium- und Kaliumcarbonat, Hydrogencarbonate wie Natrium- und Kaliumhydrogencarbonat, Oxide wie Calcium- und Magnesiumoxid oder Hydroxide wie Natrium-, Kalium-, Calcium- oder Magnesiumhydroxid; Aether wie Diäthyläther, Tetrahydrofuran, Dioxan, Aethylenglykoldimethyläther oder Diäthylenglykoldimethyläther; Ketone wie Aceton, 2-Butanon, 3-Pentanon oder Cyclohexanon; chlorierte Kohlenwasserstoffe wie Chlorbenzol, Dichlorbenzol, Methylenchlorid, Chloroform, Tetrachlorkohlenstoff, Trichloräthan oder Tetrachloräthan; sekundäre Alkohole wie 2-Butanol, Isopropanol oder Cyclohexanol; oder Kohlenwasserstoffe wie Cyclohexan, Benzol, Toluol oder Xylol. Bevorzugt ist die Umsetzung mit wässriger Ammoniak-Lösung in Gegenwart von Toluol oder Xylol.

Die Dechlorierung der Verbindung der Formel IV zur Verbindung der Formel I durch katalytische Hydrierung wird im allgemeinen unter milden Bedingungen bei Temperaturen zwischen 20°C und 70°C bei einem Druck von 1 bis 5 bar, vorzugsweise 1 bis 1,5 bar, in einem inerten Lösungsmittel in Gegenwart eines säurebindenden Mittels mit Wasserstoff durchgeführt. Als Katalysatoren werden im wesentlichen Edelmetallkatalysatoren verwendet wie Platin in Form von Platinoxid oder Palladium, Platinmoor oder Platin auf Bariumsulfat, Palladiummoor oder Palladium auf Kohle. Am breitesten einsetzbar ist Palladium auf Kohle in handelsüblicher Form als 5 % Palladium/Kohle. Als säurebindende Mittel werden üblicherweise verwendet: Hydroxide wie Natrium- oder Kaliumhydroxid, Metallsalze von Carbonsäuren wie z.B. Natriumacetat, Salze der Phosphorsäure wie z.B. Dinatriumphosphat, Hydrogencarbonat, Oxide wie Magnesium- oder Calciumoxide sowie vorzugsweise tertiäre organische Amine wie Trimethylamin, Triäthylamin, Diazabicyclo-[2.2.2]octan, 1,8-Diazabicyclo[5.4.0]undec-7-en, 1,5-Diazabicyclo[4.3.0]non-5-en, Pyridin, Chinolin oder Isochinolin. Diese Basen werden entweder während der Reaktion kontinuierlich dem Reaktionsgemisch zugefügt, wobei der pH-Wert konstant gehalten wird, oder vorzugsweise in der benötigten Menge vorgelegt. Als Lösungsmittel kommen in Frage: Aether wie Diäthyläther, Tetrahydrofuran oder Dioxan; Ketone wie Aceton, 2-Butanon oder Cyclohexanon; Ester wie Aethylacetat; Alkohole wie Methanol, Aethanol, n-Propanol, i-Propanol oder Butanol und Kohlenwasserstoffe wie Pentan, Hexan, Cyclohexan, Benzol, Toluol oder Xylol; oder Wasser. In der bevorzugten Ausführungsform wird die Verbindung der Formel IV unter Normaldruck bei 50°C bis 55°C in Gegenwart eines 5 %igen Palladium/Kohle-Katalysators in einem 2-Butanon/Wasser-Base-Gemisch mit Wasserstoff hydriert.

In einer bevorzugten Ausführungsform des erfindungsgemässen Verfahrens zur Herstellung der Verbindungen der Formel I setzt man eine Verbindung der Formel II bei einer Temperatur zwischen -20°C und +60°C im n-Dekan mit einer äquimolaren Menge Chlorsulfonsäure um, neutralisiert die Reaktionsmischung mit Alkalihydroxid bei 50°C bis 90°C, versetzt das entstandene Sulfonsäure-alkalisalz der Formel V in Xylol bei einer Temperatur zwischen 60°C und 120°C mit Phosgen; behandelt das entstandene Sulfonylchlorid der Formel III in Gegenwart von Toluol oder Xylol mit wässriger Ammoniak-Lösung und dechloriert das erhaltene Sulfonamid der Formel IV durch katalytische Hydrierung in einem 2-Butanon/Wasser-Base-Gemisch mit Wasserstoff unter Normaldruck bei einer Temperatur von 20°C bis 70°C in Gegenwart von 5 %igem Palladium/Kohle-Katalysator.

Die Zwishenprodukte der Formeln III und IV, worin R jeweils für Chlormethyl, Methoxymethyl oder $C_1$-$C_4$-Alkyl steht, sowie die Zwishenprodukte der Formel V sind neu. Sie wurden speziell für die Durchführung des erfindungsgemässen Verfahrens entwickelt und bilden daher einen Bestandteil der vorliegenden Erfindung.

Die folgenden Beispiele dienen der näheren Illustration der vorliegenden Erfindung. Das Beispiel H2 ist als Alternative zum ersten und zweiten Reaktionsschritt des Beispiels H1 zu verstehen. Das Beispiel H3 kann wahlweise den letzen Reaktionsschritt des Beispiels H1 ersetzen.

## Herstellungsbeispiele:

### Beispiel H1: 2-(2-Chloräthoxy)-benzosulfonamid

a) 2-(2-Chloräthoxy)-5-chlorbenzosulfonsäure-natriumsalz

Eine Lösung von 191 g (1,00 Mol) 4-Chlor-(2-chloräthoxy)-benzol in 300 ml n-Dekan wird bei einer Temperatur zwischen 35°C und 40°C unter kräftigem Rühren innerhalb von 2 Stunden mit 122 g (1,05 Mol) Chlorsulfonsäure versetzt. Es entwickelt sich Chlorwasserstoffgas und ein Niederschlag fällt aus.

Man erwärmt die Reaktionslösung auf +80°C, setzt 50 ml Wasser zu und neutralisiert die Mischung auf pH 7 durch Zugabe von 160 g 30%iger (1,20 Mol) Natriumhydroxidlösung. Die heisse wässrige Phase wird abgetrennt und mit 700 ml Xylol versetzt. Aus dieser Mischung wird das Wasser durch Destillation am Wasserabscheider vertrieben, wobei das salzartige Produkt aus der Xylollösung ausfällt und eine Suspension bildet. Durch Filtration und Trocknung erhält man 302 g (100 % der Theorie) 2-(2-Chloräthoxy)-5-chlorbenzosulfonsäure-natriumsalz.

In analoger Weise erhält man das entsprechende Kaliumsalz, beziehungsweise die Natrium- oder Kaliumsalze von 2-(2-Methoxyäthoxy)-5-chlorbenzosulfonsäure und 2-(2,2,2-Trifluoräthoxy)-5-chlorbenzosulfonsäure.

b) 2-(2-Chloräthoxy)-5-chlorbenzosulfonylchlorid

Die unter a) erhaltene Suspension von 302 g 2-(2-Chloräthoxy)-5-chlorbenzosulfonsäure-natriumsalz in 700 ml Xylol wird bei einer Temperatur zwischen 85°C und 90°C mit 1,5 g (0,02 Mol) Dimethylformamid versetzt. Anschliessend werden in das Gemisch innerhalb von 2 Stunden 150 g (1,51 Mol) gasförmiges Phosgen eingeleitet. Ueberschüssiges Phosgen wird durch Einleiten von Stickstoffgas für 30 Minuten vertrieben. Die Mischung wird bei 50°C mit 200 ml Wasser versetzt. Die organische Phase wird abgetrennt und eingedampft. Man erhält 287 g (99,2 % der Theorie) 2-(2-Chloräthoxy)-5-chlorbenzosulfonylchlorid mit einem Schmelzpunkt von 94,5-95°C.

In analoger Weise erhält man 2-(2-Methoxyäthoxy)-5-chlorbenzosulfonylchlorid als Oel und 2-(2,2,2-Trifluoräthoxy)-5-chlorbenzosulfonylchlorid, Smp. 76°C.

c) 2-(2-Chloräthoxy)-5-chlorbenzosulfonamid

Die unter b) erhaltene Lösung vom 287 g 2-(2-Chloräthoxy)-5-chlorbenzosulfonylchlorid in 700 ml Xylol wird bei einer Temperatur zwischen 55°C und 60°C innerhalb von 2 Stunden mit 142 g 30%iger (2,5 Mol) Ammoniak-Lösung versetzt. Es bildet sich ein farbloser kristalliner Niederschlag. Die Temperatur wird für weitere zwei Stunden bei 55°C gehalten und anschliessend auf 0°C gesenkt. Das Kristallisat wird abgetrennt und dreimal mit Isopropanol gewaschen (einmal mit 150 ml, zweimal mit je 500 ml). Nach Trocknung des Produkts beträgt die Ausbeute an 2-(2-Chloräthoxy)-5-chlorbenzosulfonamid 250 g (92,6 % d. Th.), Smp. 145°C.

In analoger Weise erhält man 2-(2-Methoxyäthoxy)-5-chlorbenzosulfonamid, Smp. 121-123°C, und 2-(2,2,2-Trifluoräthoxy)-5-chlorbenzosulfonamid, Smp. 142,5°C.

d) In einem Rührkolben wird ein Gemisch aus 67,5 g (0,25 Mol) 2-(2-Chloräthoxy)-5-chlorbenzosulfonamid, 165 g 2-Butanon, 36 g Wasser, 3,4 g 5 % Palladium/Kohle und 0,22 g Essigsäure bei einer Temperatur zwischen 50°C und 55°C unter Normaldruck mit Wasserstoff hydriert, Während der Hydrierungsreaktion wird der pH-Wert durch Zutropfen von 33,5 g (0,25 Mol) 30%iger Natriumhydroxidlösung konstant zwischen pH 10,5 und pH 11,0 gehalten. Die Reaktionszeit bis zur Sättigung mit Wasserstoff beträgt 45 Minuten. Das Produkt wird durch Filtration und Einengen des Filtrats gewonnen. Man erhält 58,2 g (99 % d. Th.) 2-(2-Chloräthoxy)-benzosulfonamid mit einem Schmelzpunkt von 119°C.

In analoger Weise erhält man 2-(2-Methoxyäthoxy)-benzosulfonamid, Smp. 111°C, und 2-(2,2,2-Trifluoräthoxy)-benzosulfonamid, Smp. 123°C.

Beispiel H2: 2-(2-Chloräthoxy)-5-chlorbenzosulfonychlorid

Zu einer Lösung von 233 g (2 Mol) Chlorsulfonsäure in 105 ml 1,2-Dichloräthan lässt man bei einer Temperatur von +5°C unter Rühren innerhalb von 0,5 Stunden 64,0 g (0,33 Mol) 4-Chlor-(2-chloräthoxy)-benzol zutropfen. Die Reaktionsmischung wird für weitere 1,5 Stunden bei 20°C gerührt und dann in einem Gemisch aus 230 g Eis, 170 ml Wasser und 60 ml 1,2-Dichloräthan aufgenommen. Die organische Phase wird abgetrennt, mit Wasser gewaschen und eingedampft. Man erhält so 77 g (80 % d. Th.) 2-(2-Chloräthoxy)-5-chlorbenzosulfonychlorid.

In analoger Weise erhält man auch 2-n-Propoxy-5-chlorbenzolsulfonylchlorid, 2-Aethoxy-5-chlorbenzolsulfonylchlorid, 2-(2-Methoxyäthoxy)-5-chlorbenzosulfonylchlorid und 2-(2,2,2-Trifluoräthoxy)-5-chlorbenzosulfonylchlorid.

Beispiel H3: 2-(2-Chloräthoxy)-benzosulfonamid

In einem Rührkolben werden 68,2 g 2-(Chloräthoxy)-5-chlorbenzosulfonamid in 184 g 2-Butanon gelöst. Nach Zugabe von 34,3 g Wasser, 34,3 g 20 %iger Natriumhydroxidlösung und 3,4 g 5 % Palladium/Kohle wird bei 25-30°C unter Normaldruck mit Wasserstoff hydriert. Die Hydrierdauer beträgt 30 Minuten. Der Katalysator wird abfiltriert und das Filtrat eingeengt. Man erhält 58,7 g (98 % d.Th.) 2-(2-Chloräthoxy)-benzosulfonamid mit einem Schmelzpunkt von 119°C.

In analoger Weise erhält man 2-(2-Methoxyäthoxy)-benzosulfonamid und 2-(2,2,2-Trifluoräthoxy)-benzosulfonamid.

**Patentansprüche**

**1.** Verfahren zur Herstellung von 2-Alkoxybenzosulfonamiden der Formel I

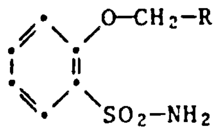

(I)

worin R für Chlormethyl, Methoxymethyl, $C_1$-$C_4$ Alkyl oder Trifluormethyl steht, dadurch gekennzeichnet, dass man ein 4-Alkoxy-chlorbenzol der Formel II

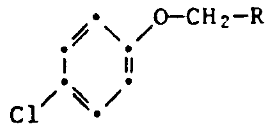

(II)

worin R die unter Formel I gegebene Bedeutung hat, mit Chlorsulfonsäure $ClSO_3H$ umsetzt, das entstandene Sulfonylchlorid der Formel III

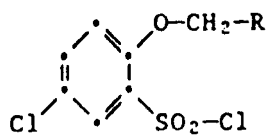

(III)

worin R die unter Formel I gegebene Bedeutung hat, mit Ammoniak in das Sulfonamid der Formel IV

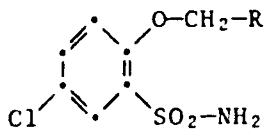

(IV)

worin R die unter Formel I gegebene Bedeutung hat, überführt und dieses in Gegenwart eines Edelmetallkatalysators mit Wasserstoff hydriert.

**2.** Verfahren gemäss Anspruch 1, dadurch gekennzeichnet, dass man die Umsetzung von Verbindung II zur Verbindung III in zwei Stufen durchführt, indem man die Verbindung II mit einem Aequivalent Chlorsulfonsäure umsetzt, das Reaktionsgemisch mit wässrigem Alkalihydroxid neutralisiert und das entstandene Sulfonsäure-alkalisalz der Formel V

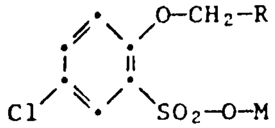

(V)

worin R die unter Formel I gegebene Bedeutung hat und M für ein Natrium- oder Kaliumatom steht, mit einem Chlorierungsmittel in das Sulfonylchlorid der Formel III umwandelt.

**3.** Verfahren gemäss Anspruch 1, dadurch gekennzeichnet, dass man die Reaktion in allen Reaktionsstufen bei Temperaturen zwischen -10 °C und +100 °C, vorzugsweise zwischen -10 °C und +80 °C, durchführt.

**4.** Verfahren gemäss Anspruch 1, dadurch gekennzeichnet, dass man die einzelnen Reaktionsstufen in inerten Lösungsmitteln durchführt.

**5.** Verfahren gemäss Anspruch 4, dadurch gekennzeichnet, dass man die Reaktion der Verbindung II zur Verbindung III in Methylenchlorid, 1,2-Dichloräthan oder Chloroform; die Reaktion der Verbindung III zur Verbindung IV in Wasser in Gegenwart von Toluol oder Xylol und die Reaktion der Verbindung IV zur Verbindung I in einem 2-Butanon/Wasser-Gemischausführt.

**6.** Verfahren gemäss Anspruch 1, dadurch gekennzeichnet, dass man die Umsetzung der Verbindung der Formel II zur Verbindung der Formel III in einem inerten Lösungsmittel bei einer Temperatur zwischen -10 °C und +100 °C mit mindestens 3 Mol Chlorsulfonsäure pro Mol der Verbindung der Formel II durchführt.

**7.** Verfahren gemäss Anspruch 6, dadurch gekennzeichnet, dass man die Umsetzung der Verbindung der Formel II zur Verbindung der Formel III bei einer Temperatur zwischen -10 °C und +80 °C in Methylenchlorid, 1,2-Dichloräthan oder Chloroform mit mindestens 5 Mol Chlorsulfonsäure pro Mol der Verbindung der Formel II durchführt.

**8.** Verfahren gemäss Anspruch 2, dadurch gekennzeichnet, dass man die Umsetzung der Verbindung der Formel III zur Verbindung der Formel IV in wässriger Ammoniak-Lösung in Gegenwart von Toluol oder Xylol durchführt.

**9.** Verfahren gemäss Anspruch 2, dadurch gekennzeichnet, dass man die Dechlorierung der Verbindung der Formel IV zur Verbindung der Formel IV bei einem Druck zwischen 1 und 5 bar bei 20 °C bis 70 °C in Gegenwart eines 5 %igen Palladium/Kohle-Katalysators in einem 2-Butanon/Wasser-Base-Gemisch mit Wasserstoff durchführt.

**10.** Verfahren gemäss Anspruch 2, dadurch gekennzeichnet, dass man die Verbindung der Formel II bei einer Temperatur zwischen 20 °C und 60 °C mit einem Aequivalent Chlorsulfonsäure in Cyclohexan oder n-Dekan umsetzt, die Lösung mit wässrigem Alkalihydroxid bei einer Temperatur von zwischen 50 °C und 90 °C neutralisiert und das Sulfonsäure-alkalisalzder Formel V bei einer Temperatur zwischen 60 °C und +120 °C mit Phosgen in das Sulfonylchlorid der Formel III überführt.

**11.** Verfahren gemäss Anspruch 2, dadurch gekennzeichnet, dass man eine Verbindung der Formel II bei einer Temperatur zwischen 20 °C und +60 °C in n-Dekan mit einer äquimolaren Menge Chlorsulfonsäure umsetzt, die Reaktionsmischung bei einer Temperatur zwischen 50 °C und 90 °C mit Alkalihydroxid neutralisiert und das entstandene Sulfonsäure-alkalisalz der Formel V in Xylol bei einer Temperatur zwischen 60 °C und 120 °C mit Phosgen versetzt, das entstandene Sulfonylchlorid der Formel III in Gegenwart von Toluol oder Xylol mit wässriger Ammoniak-Lösung behandelt und das erhaltene Sulfonamid der Formel IV durch katalytische Hydrierung in einem 2-Butanon/Wasser-Base-Gemisch mit Wasserstoff unter Normaldruck bei einer Temperatur von 20 °C bis 70 °C in Gegenwart von 5 %igem Palladium/Kohle-Katalysator dechloriert.

**12.** 2-Alkoxy-5-chlorbenzosulfonylchloride der Formel III

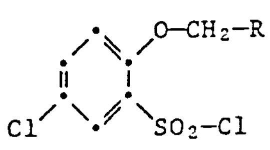

(III)

worin R für Chlormethyl, Methoxymethyl oder $C_1$-$C_4$-Alkyl steht.

**13.** 2-Alkoxy-5-chlorbenzosulfonamide der Formel IV

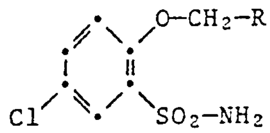

(IV)

worin R für Chlormethyl, Methoxymethyl oder $C_1$-$C_4$-Alkyl steht.

**14.** 2-Alkoxy-5-chlorbenzosulfonsäurealkalisalz der Formel V

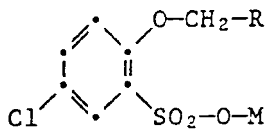

(V)

worin R für Chlormethyl, Methoxymethyl, $C_1$-$C_4$-Alkyl oder Trifluormethyl und M für ein Natrium- oder Kaliumatom steht.

**Claims**

**1.** A process for the preparation of a 2-alkoxybenzenesulfonamide of formula I

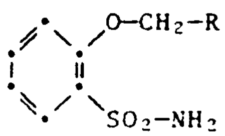

(I)

wherein R is chloromethyl, methoxymethyl, $C_1$-$C_4$alkyl or trifluoromethyl, which process comprises reacting a 4-alkoxychlorobenzene of formula II

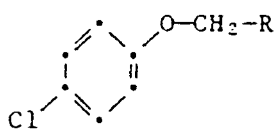

(II)

wherein R is as defined for formula I, with chlorosulfonic acid $ClSO_3H$, converting the resultant sulfonyl chloride of formula III

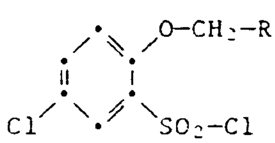

(III)

wherein R is as defined for formula I, by reaction with ammonia into the sulfonamide of formula IV

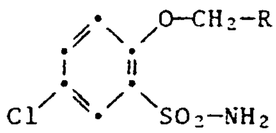

(IV)

wherein R is as defined for formula I, and hydrogenating said sulfonamide with hydrogen, in the presence of a noble metal catalyst.

**2.** A process according to claim 1, wherein the reaction of the compound of formula II to give the compound of formula III is carried out in two steps by reacting the compound of formula II with one equivalent of chlorosulfonic acid, neutralising the reaction mixture with an aqueous solution of alkali metal hydroxide and converting the resultant sulfonic acid alkali metal salt of formula V

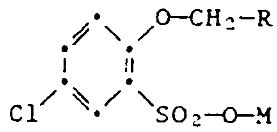

(V)

wherein R is as defined for formula I and M is a sodium or potassium atom, by reaction with a chlorinating agent into the sulfonyl chloride of formula III.

**3.** A process according to claim 1, wherein the reaction in all its stages is carried out in the temperature range from -10 °C to +100 °C, preferably from -10 °C to +80 °C.

**4.** A process according to claim 1, wherein the individual reaction steps are carried out in inert solvents.

**5.** A process according to claim 4, wherein the reaction of the compound of formula II to give the compound of formula III is carried out in methylene chloride, 1,2-dichloroethane or chloroform; the reaction of the compound of formula III to give the compound of formula IV is carried out in water and in the presence of toluene or xylene; and the reaction of the compound of formula IV to give the compound of formula I is carried out in a mixture of 2-butanone and water.

**6.** A process according to claim 1, wherein the reaction of the compound of formula II to give the compound of formula III is carried out with at least 3 moles of chlorosulfonic acid per mole of the compound of formula II, in an inert solvent and at a temperature in the range from -10 °C to +100 °C.

**7.** A process according to claim 6, wherein the reaction of the compound of formula II to give the compound of formula III is carried out with at least 5 moles of chlorosulfonic acid per mole of the compound of formula II, at a temperature in the range from -10 °C to +80 °C, in methylene chloride, 1,2-dichloroethane or chloroform.

**8.** A process according to claim 2, wherein the reaction of the compound of formula III to give the compound of formula IV is carried out in an aqueous solution of ammonia and in the presence of toluene or xylene.

**9.** A process according to claim 2, wherein the dechlorination of the compound of formula IV to give the compound of formula IV is carried out with hydrogen, in the pressure range from 1 to 5 bar, at a temperature in the range from +20 °C to +70 °C and in the presence of a 5 % palladium on carbon catalyst in a mixture of 2-butanone, base and water.

**10.** A process according to claim 2, wherein the compound of formula II is reacted, at a temperature in the range from +20 °C to +60 °C, with 1 equivalent of chlorosulfonic acid in cyclohexane or n-decane; the solution is neutralised with aqueous alkali metal hydroxide, at a temperature in the range from +50 °C to +90 °C; and the sulfonic acid alkali metal salt of formula V is converted, at a temperature in the

range from +60°C to +120°C, by reaction with phosgene into the sulfonyl chloride of formula III.

**11.** A process according to claim 2, wherein a compound of formula II is reacted, at a temperature in the range from +20°C to +60°C and in n-decane, with an equimolar amount of chlorosulfonic acid; the reaction mixture is neutralised with alkali metal hydroxide, at a temperature in the range from +50°C to +90°C; phosgene is added to the resultant sulfonic acid alkali metal salt of formula V, in xylene and at a temperature in the range from +60°C to +120°C; the resultant sulfonyl chloride of formula III is treated with an aqueous solution of ammonia, in the presence of toluene or xylene; and the resultant sulfonamide of formula IV is dechlorinated by catalytic hydrogenation with hydrogen, in a mixture of 2-butanone base and water, under normal pressure, at a temperature in the range from +20°C to +70°C and in the presence of a 5 % palladium on carbon catalyst.

**12.** A 2-alkoxy-5-chlorobenzenesulfonyl chloride of formula III

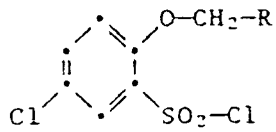

(III)

wherein R is chloromethyl, methoxymethyl or $C_1$-$C_4$ alkyl.

**13.** A 2-alkoxy-5-chlorobenzenesulfonamide of formula IV

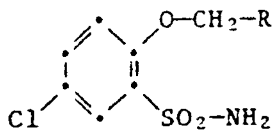

(IV)

wherein R is chloromethyl, methoxymethyl or $C_1$-$C_4$ alkyl.

**14.** A 2-alkoxy-5-chlorobenzenesulfonic acid alkali metal salt of formula V

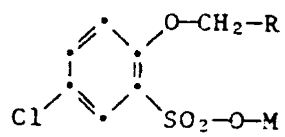

(V)

wherein R is chloromethyl, methoxymethyi, $C_1$-$C_4$alkyl or trifluoromethyl and M is a sodium or potassium atom.

## Revendications

**1.** Procédé pour la préparation des 2-alcoxybenzosulfonamides de formule I

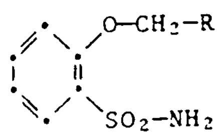

(I)

où R représente le chlorométhyle, le méthoxyméthyle, un alkyle en $C_1$-$C_4$ ou le trifluorométhyle, caractérisé en ce que l'on fait réagir un 4-alcoxy-chlorobenzène de formule II

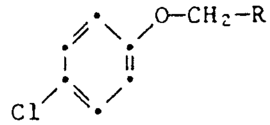

(II)

où R a la signification donnée sous la formule I, avec l'acide chlorosulfonique $ClSO_2H$, en ce que l'on transforme le chlorure de sulfonyle formé de formule III

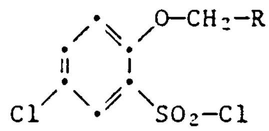

(III)

où R a la signification donnée sous la formule I, avec l'ammoniac en sulfonamide de formule IV

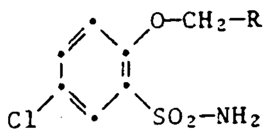

(IV)

où R a la signification donnée sous la formule I et en ce que l'on hydrogène celui-ci avec l'hydrogène en presence d'un catalyseur à base d'un métal noble.

**2.** Procédé selon la revendication 1, caractérisé en ce que l'on effectue la transformation du composé II en composé III en deux étapes, en faisant réagir le composé II avec un équivalent d'acide chlorosulfonique, en neutralisant le mélange réactionnel avec un hydroxyde alcalin aqueux et en transformant le sel alcalin de l'acide sulfonique obtenu de formule V

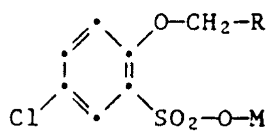

(V)

où R a la signification donnée sous la formule I et M représente un atome de sodium ou de potassium, avec un agent de chloration en chlorure de sulfonyle de formule III.

**3.** Procédé selon la revendication 1, caractérisé en ce que l'on effectue la réaction, dans toutes les étapes réactionnelles, à des températures entre -10 ° C et + 100 ° C, de préférence, entre -10 ° C et + 80 ° C.

**4.** Procédé selon la revendication 1, caractérisé en ce que l'on effectue les différentes étapes réactionnelles dans des solvants inertes.

**5.** Procédé selon la revendication 4, caractérisé en ce que l'on effectue la réaction conduisant du composé II au composé III dans le chlorure de méthylène, le 1,2-dichloroéthane ou le chloroforme ; la réaction conduisant du composé III au composé IV dans l'eau en présence de toluène ou de xylène et la réaction conduisant du composé IV au composé I dans un mélange 2-butanone/eau.

**6.** Procédé selon la revendication 1, caractérisé en ce que l'on effectue la transformation du composé de formule II en composé de formule III, dans un solvant inerte à une température entre -10 ° C et + 100 ° C, avec au moins 3 moles d'acide chlorosulfonique par mole de composé de formule II.

**7.** Procédé selon la revendication 6, caractérisé en ce que l'on effectue la transformation du composé de formule II en composé de formule III à une température entre -10°C et +80°C dans le chlorure de méthylène, le 1,2-dichloroéthane ou le chloroforme avec au moins 5 moles d'acide chlorosulfonique par mole de composé de formule II.

**8.** Procédé selon la revendication 2, caractérisé en ce que l'on effectue la transformation du composé de formule III en composé de formule IV dans une solution aqueuse ammoniacale en présence de toluène ou de xylène.

**9.** Procédé selon la revendication 2, caractérisé en ce que l'on effectue la déchloration du composé de formule IV pour obtenir le composé de formule I sous une pression entre 1 et 5 bar à une température allant de 20°C à 70°C en présence d'un catalyseur palladium/charbon à 5% dans un mélange base 2-butanone/eau avec de l'hydrogène.

**10.** Procédé selon la revendication 2, caractérisé en ce que l'on fait réagir le composé de formule II à une température entre 20°C et 60°C avec un équivalent d'acide chlorosulfonique dans le cyclohexane ou le n-décane, en ce que l'on neutralise la solution avec un hydroxyde alcalin aqueux à une température entre 50°C et 90°C et en ce que l'on transforme le sel alcalin de l'acide sulfonique de formule V à une température entre 60°C et +120°C par le phosgène en chlorure de sulfonyle de formule III.

**11.** Procédé selon la revendication 2, caractérisé en ce que l'on fait réagir un composé de formule II à une température entre 20°C et +60°C dans le n-décane avec une quantité équimolaire d'acide chlorosulfonique, en ce que l'on neutralise le mélange réactionnel à une température entre 50°C et 90°C avec un hydroxyde alcalin, en ce que l'on additionne le sel alcalin de l'acide sulfonique de formule V obtenu dans le xylène à une température entre 60°C et 120°C de phosgène, en ce que l'on traite le chlorure de sulfonyle de formule III obtenu en présence de toluène ou de xylène avec une solution aqueuse ammoniacale et en ce que l'on déchlore le sulfonamide de formule IV obtenu, par hydrogénation catalytique, dans un mélange base 2-butanone/eau par l'hydrogène à une pression normale à une température allant de 20°C à 70°C en présence d'un catalyseur palladium/charbon à 5%.

**12.** Les chlorures de 2-alcoxy-5-chlorobenzosulfonyle de formule III

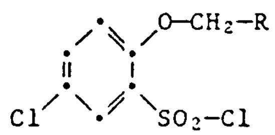

(III)

où R représente le chlorométhyle, le méthoxyméthyle ou un alkyle en $C_1$-$C_4$.

**13.** Les 2-alcoxy-5-chlorobenzosulfonamides de formule IV

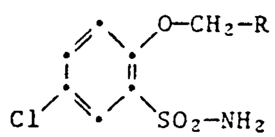

(IV)

où R représente le chlorométhyle, le méthoxyméthyle ou un alkyle en $C_1$-$C_4$.

**14.** Le sel alcalin d'un acide 2-alcoxy-5-chlorobenzosulfonique de formule V

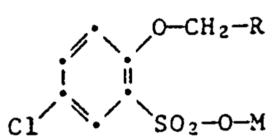

(V)

où R représente le chlorométhyle, le méthoxyméthyle, un alkyle en $C_1$-$C_4$ ou le trifluorométhyle et M représente un atome de sodium ou de potassium.